# EUROPÄISCHE PATENTSCHRIFT

(11) **EP 4 058 089 B1**
(45) Veröffentlichungstag und Bekanntmachung des Hinweises auf die Patenterteilung: **06.05.2026**
(21) Anmeldenummer: 20804259.8
(22) Anmeldetag: 10.11.2020
(51) Int. Cl.: A61M 1/16, A61M 1/34

(54) **SIGNALGETRIGGERTES VERFAHREN ZUM ENTLEEREN EINES EFFLUENTBEUTELS UND VORRICHTUNGEN**
SIGNAL-TRIGGERED METHOD FOR EVACUATING AN EFFLUENT BAG AND DEVICES
PROCÉDÉ D'ÉVACUATION D'UN SAC D'EFFLUENT DÉCLENCHÉ PAR UN SIGNAL ET DISPOSITIFS

(30) Priorität: 12.11.2019 DE 102019130434
(43) Veröffentlichungstag der Anmeldung: 21.09.2022
(73) Patentinhaber: Fresenius Medical Care Deutschland GmbH, 61352 Bad Homburg (DE)
(72) Erfinder: KLEWINGHAUS, Juergen, 61440 Oberursel (DE)
(74) Vertreter: Bobbert & Partner Patentanwälte PartmbB
(86) Internationale Anmeldenummer: PCT/EP2020/081603
(87) Internationale Veröffentlichungsnummer: WO 2021/094294

(56) Entgegenhaltungen:
- EP-A1- 2 338 542
- CA-A1- 3 069 257
- DE-A1- 102017 127 394
- US-A1- 2011 036 768
- US-A1- 2017 141 601

## Beschreibung

Die vorliegende Erfindung betrifft eine Steuer- oder Regelvorrichtung gemäß Anspruch 1, eine Behandlungsvorrichtung gemäß Anspruch 6 und eine Effluentbeutelentleerungsvorrichtung gemäß Anspruch 8. Ferner betrifft sie ein System gemäß Anspruch 11. Die Offenbarung betrifft außerdem ein digitales Speichermedium, ein Computerprogramm-Produkt, ein Computerprogramm sowie ferner ein Verfahren zum Entleeren eines Effluentbeutels, bzw. gemäß jeweils der Oberbegriffe oder Gattungsbegriffe dieser Ansprüche.

Aus der Praxis ist die extrakorporale Blutbehandlung bekannt. Dabei wird dem Patienten Blut entnommen, das entlang eines Blutkreislaufs extrakorporal und z. B. durch einen Blutfilter geführt wird. Der Blutfilter weist eine Blutkammer, durch welche Blut geführt wird, und eine Dialysierflüssigkeitskammer, durch welche Dialysierflüssigkeit geführt wird, auf. Beide Kammern sind durch eine semi-permeable Membran voneinander getrennt. Blut und Dialysierflüssigkeit werden zumeist im Gegenstromprinzip durch den Blutfilter geleitet. Das Blut wird im Blutfilter gereinigt, die Dialysierflüssigkeit gilt bei ihrem Austritt aus dem Blutfilter als verbraucht und wird, nun als Dialysat bezeichnet, verworfen. Neben dem Dialysat umfasst das zu verwerfende Fluid auch Filtrat, welches Wasser, das dem Blut im Blutfilter entzogen wurde, umfasst. Filtrat und Dialysat werden im Folgenden einzeln oder gemeinsam vereinfacht als Effluent bezeichnet.

Das Effluent wird in der Praxis mittels einer Effluentablaufleitung unmittelbar verworfen oder, vor allem bei der Akutbehandlung, einem Effluentbeutel zugeführt und darin zunächst aufbewahrt. Nach Beendigung der Blutbehandlung, oder in während der Blutbehandlung liegenden Beutelleerintervallen (Intervalle, in welchen der Effluentbeutel geleert wird), wird das Effluent aus dem Effluentbeutel, z. B. in ein Waschbecken oder einen anders gestalteten Ausguss verworfen. Der relevante Stand der Technik ist aus den Dokumenten DE102017127394, CA3069257, US2011/036768 und EP2338542 bekannt.

Eine Aufgabe der vorliegenden Erfindung kann darin gesehen werden, eine Steuer- oder Regelvorrichtung, eine Blutbehandlungsvorrichtung und eine Effluentbeutelentleerungsvorrichtung anzugeben, weiterhin ein System aus oder mit den beiden letztgenannten Vorrichtungen.

Außerdem sollen ein digitales Speichermedium, ein Computerprogramm-Produkt sowie ein Computerprogramm angegeben werden.

Ferner soll ein Verfahren zum Entleeren von Effluentbeuteln angegeben werden, das mit dem oben genannten System durchführbar ist.

Die erfindungsgemäße Aufgabe wird durch eine Steuer- oder Regelvorrichtung mit den Merkmalen des Anspruchs 1, eine Behandlungsvorrichtung mit den Merkmalen des Anspruchs 6 und eine Effluentbeutelentleerungsvorrichtung mit den Merkmalen des Anspruchs 8 gelöst. Zudem wird sie gelöst durch ein System mit den Merkmalen des Anspruchs 11.

Die Erfindung wird durch die beigefügten Ansprüche definiert.

Die vorliegende Erfindung betrifft somit eine Steuer- oder Regelvorrichtung, welche zum Steuern oder Regeln einer Blutbehandlungsvorrichtung zum Behandeln des Bluts eines Patienten programmiert ist. Die Behandlung erfolgt im Rahmen einer Behandlungssitzung, die optional unter Verwenden eines extrakorporalen Blutschlauchsatzes und unter Befüllen eines Effluentbeutels mit Effluent durchgeführt wird. Während der Behandlungssitzung werden vorzugsweise Flüssigkeitsströme (z. B. die Ströme von Calciumlösung, Citratlösung, Substituatlösung (in Prädilution und/oder in Postdilution), Heparinlösung, Netto-Ultrafiltrationsrate, Dialysatflussrate, jeweils falls zutreffend oder gegeben) bilanziert, welche mittels einer Calciumpumpe, einer Citratpumpe, einer Substituatpumpe, einer Heparinpumpe, einer Filtratpumpe und/oder mittels einer Dialysierflüssigkeitspumpe der Blutbehandlungsvorrichtung erzeugt werden.

Die erfindungsgemäße Steuer- oder Regelvorrichtung ist weiter programmiert, um die Bilanzierung, den Fluss mittels einer oder mehreren, beliebig kombinierbaren, der verwendeten Pumpen, insbesondere der vorstehend genannten, z. B. den Fluss der Calciumlösung (oder die Fördertätigkeit der Calciumpumpe), der Citratlösung (oder die Fördertätigkeit der Citratpumpe), der Heparinlösung (oder die Fördertätigkeit der Heparinpumpe), den Netto-Ultrafiltratfluss (oder die Fördertätigkeit der Filtrationspumpe), den Substituatfluss (oder die Fördertätigkeit der Substituatpumpe) und/oder den Dialysierflüssigkeitsfluss (oder die Fördertätigkeit der Dialysierflüssigkeitspumpe) während der Blutbehandlungssitzung und/oder innerhalb der Dauer der Blutbehandlungssitzung, vorübergehend unterbrechen und wieder starten zu können.

Die Steuer- oder Regeleinrichtung ist insbesondere programmiert, ein solches Unterbrechen, vorzugsweise automatisch, also ohne menschliches Zutun, zu einem oder zu mehreren vorbestimmten Unterbrechungszeitpunkten zu veranlassen, die während der Behandlungssitzung, also innerhalb der Dauer der Behandlung, liegen.

Die erfindungsgemäße Blutbehandlungsvorrichtung umfasst eine solche erfindungsgemäße Steuer- oder Regelvorrichtung.

Die erfindungsgemäße Effluentbeutelentleerungsvorrichtung umfasst eine Effluentablaufleitung, die sich mit einem Effluentbeutel in Fluidverbindung verbinden lässt, und die hierin auch als Ausgussleitung bezeichnet werden könnte, zum Leiten von Effluent aus einem Effluentbeutel heraus. Die Effluentbeutelentleerungsvorrichtung umfasst außerdem einen Pumpenabschnitt zum aktiven Fördern von Effluent entlang der Effluentablaufleitung und damit aus dem Effluentbeutel heraus.

Eine Steuervorrichtung (die optional konfiguriert ist, um auch zu regeln) ist ebenfalls von der erfindungsgemäßen Effluentbeutelentleerungsvorrichtung umfasst. Die Steuervorrichtung ist programmiert, um den Pumpenabschnitt zu veranlassen, Effluent aus dem Effluentbeutel herauszufördern, wenn (oder sobald) ein vorbestimmter Entleerungszeitpunkt eintritt.

Das erfindungsgemäße System umfasst eine erfindungsgemäße Blutbehandlungsvorrichtung und eine erfindungsgemäße Effluentbeutelentleerungsvorrichtung.

Das digitale Speichermedium ist konfiguriert, um derart mit einem programmierbaren Computersystem zusammenzuwirken, dass eine Steuer- oder Regelvorrichtung einer Blutbehandlungsvorrichtung zu einer erfindungsgemäßen Steuer- oder Regelvorrichtung umprogrammiert wird, wodurch die Blutbehandlungsvorrichtung zu einer erfindungsgemäßen Blutbehandlungsvorrichtung wird, und/oder dass eine Steuervorrichtung einer Effluentbeutelentleerungsvorrichtung derart programmiert wird, dass die Effluentbeutelentleerungsvorrichtung zu einer erfindungsgemäßen Effluentbeutelentleerungsvorrichtung wird.

Das digitale Speichermedium kann insbesondere in Form einer Diskette, CD oder DVD, EPROM, FRAM oder SSD, mit elektronisch auslesbaren Steuersignalen, vorgesehen sein.

Das Computerprogramm-Produkt kann als Signalwelle oder mit einem auf einem maschinenlesbaren Träger gespeicherten Programmcode ausgestaltet sein, um derart mit einem programmierbaren Computersystem zusammenzuwirken, dass eine Steuer- oder Regelvorrichtung zu einer erfindungsgemäßen Steuer- oder Regelvorrichtung umprogrammiert wird und/oder dass eine Steuervorrichtung zu einer Steuervorrichtung einer erfindungsgemäßen Effluentbeutelentleerungsvorrichtung umprogrammiert wird.

Das Computerprogramm umfasst einen Programmcode, der veranlasst oder ausführt, dass eine Steuer- oder Regelvorrichtung zu einer erfindungsgemäßen Steuer- oder Regelvorrichtung umprogrammiert wird und/oder dass eine Steuervorrichtung zu einer Steuervorrichtung einer erfindungsgemäßen Effluentbeutelentleerungsvorrichtung umprogrammiert wird, wenn das Computerprogramm auf einem Computer abläuft.

Das Verfahren zum Entleeren eines Effluentbeutels, welcher während einer Blutbehandlung eines Patienten mit Effluent befüllt wurde, läuft ab an einer erfindungsgemäßen Blutbehandlungsvorrichtung und einer erfindungsgemäßen Effluentbeutelentleerungsvorrichtung bzw. an einem erfindungsgemäßen System, wobei ein Effluentbeutel, welcher eine Effluentauslassöffnung aufweist, an der Blutbehandlungsvorrichtung oder an der Effluentbeutelentleerungsvorrichtung vorgesehen ist.

Dabei ist die Effluentauslassöffnung des Effluentbeutels mit der Effluentablaufleitung der Effluentbeutelentleerungsvorrichtung verbunden, um sofort oder später Effluent aus dem Effluentbeutel herauszuleiten. Sollte ein Absperrelement für die Effluentauslassöffnung vorgesehen sein, so kann durch Öffnen des Absperrelements eine Fluidverbindung zwischen dem Inneren des Effluentbeutels und dem Inneren der Effluentablaufleitung hergestellt werden. Das Verfahren umfasst ein Anhalten oder Unterbrechen der Bilanzierung, des Ultrafiltratflusses (oder der Fördertätigkeit) der wenigstens einen Filtratpumpe, des Substituatflusses (oder der Fördertätigkeit) der Substituatpumpe und/oder des Dialysierflüssigkeitsflusses (oder der Fördertätigkeit) der Dialysierflüssigkeitspumpe oder einer der anderen hierin genannten Pumpen zu (oder ab) einem oder mehreren vorbestimmten Unterbrechungszeitpunkten, die während der Behandlungssitzung, also innerhalb der Behandlungsdauer, liegen.

Das Verfahren umfasst weiter ein Veranlassen des Pumpenabschnitts, Effluent aus dem Effluentbeutel heraus zu fördern, wenn (oder sobald) ein vorbestimmter und/oder definierter Entleerungszeitpunkt eintritt. Das Veranlassen dieser beiden Schritte (Anhalten bei Eintreten eines Unterbrechungszeitpunkts bzw. Veranlassen des Förderns bei Eintreten eines Entleerungszeitpunkts) erfolgt optional automatisch, z. B. gestartet durch oder getriggert von der Steuer- und Regelvorrichtung der Behandlungsvorrichtung und/oder von der Steuervorrichtung der Effluentbeutelentleerungsvorrichtung.

Erfindungsgemäße Ausführungsformen können manche, einige oder alle der folgenden Merkmale in beliebiger Kombination aufweisen, soweit dies für den Fachmann nicht erkennbar technisch unmöglich ist. Vorteilhafte Weiterbildungen der vorliegenden Erfindung sind jeweils auch Gegenstand der Unteransprüche.

Bei allen vorstehenden oder folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Wenn hierin von einer Ausführungsform die Rede ist, so stellt dies eine erfindungsgemäße, beispielhafte Ausführungsform dar.

Wenn hierin offenbart ist, dass der erfindungsgemäße Gegenstand ein oder mehrere Merkmale in einer bestimmten Ausführungsform aufweist, so ist hierin jeweils auch offenbart, dass der erfindungsgemäße Gegenstand genau dieses oder diese Merkmale in anderen, ebenfalls erfindungsgemäßen Ausführungsformen ausdrücklich nicht aufweist, z. B. im Sinne eines Disclaimers. Für jede hierin genannte Ausführungsform gilt somit, dass die gegenteilige Ausführungsform, beispielsweise als Negation formuliert, ebenfalls offenbart ist.

In manchen Ausführungsformen erfolgt das Unterbrechen durch die erfindungsgemäße Steuer- oder Regelvorrichtung, oder von ihr veranlasst, jeweils für eine vorbestimmte Unterbrechungszeitdauer (oder: Unterbrechungsintervall), die jeweils mit Eintreten des sie einleitenden Unterbrechungszeitpunkts beginnt. Aufeinanderfolgende Unterbrechungszeitdauern sind vorzugsweise von konstanter Länge, können aber alternativ unterschiedlich lang sein.

In einigen Ausführungsformen kann bei Eintreten des vorbestimmten Unterbrechungszeitpunkts ein Hinweis oder Alarm an den Benutzer ergehen, der ihn zum manuellen Leeren des Effluentbeutels auffordert, beispielsweise ein Hinweis auf dem Display und/oder ein optischer und/oder akustischer Alarm (Lichtquelle, Lampe, Lautsprecher, etc.).

Der Effluentbeutel steht während der Blutbehandlungssitzung mit der Blutbehandlungsvorrichtung in Fluidverbindung und nimmt während der Behandlungssitzung angefallenes Effluent auf. Er kann hierzu optional mit der Blutbehandlungsvorrichtung und/oder mit der Effluentbeutelentleerungsvorrichtung fluidisch verbunden sein. Er kann hierzu von der Blutbehandlungsvorrichtung und/oder von der Effluentbeutelentleerungsvorrichtung gehalten, getragen oder aufgenommen sein.

In manchen Ausführungsformen steht die erfindungsgemäße Steuer- oder Regelvorrichtung der Blutbehandlungsvorrichtung in Signalverbindung mit einem Signalgeber oder mit einer Uhr, z. B. einer internen Uhr (also einer Uhr der Steuer- oder Regelvorrichtung). Die Uhr kann alternativ eine externe Uhr sein, insbesondere eine Uhr der Blutbehandlungsvorrichtung, eine Netzwerkuhr, eine Stationsuhr oder eine Atomuhr. Die Steuer- oder Regelvorrichtung ist hierbei konfiguriert, um bei Erhalten eines vorbestimmten Signals vom Signalgeber oder bei Erreichen einer vorbestimmten Uhrzeit auf der Uhr den Zeitpunkt des Erhalts des Signals oder das Erreichen der vorbestimmten Uhrzeit als Unterbrechungszeitpunkt (Beginn des Unterbrechungsintervalls) zu definieren oder festzusetzen.

Die Steuer- oder Regelvorrichtung kann ebenso wie die nachfolgend beschriebene Steuervorrichtung der Effluentbeutelentleerungsvorrichtung programmiert sein, auf ein Signal des Signalgebers hin oder zu einem vorbestimmten Zeitpunkt (z. B. gemessen an einer der hierin genannten Uhren) eine Unterbrechungszeitdauer (oder -intervall) bzw. im Falle der Effluentbeutelentleerungsvorrichtung eine Entleerungsdauer (oder -intervall) sofort zu beginnen oder nach Verstreichen einer vorbestimmten Dauer oder Karenz- oder Wartedauer zu beginnen.

In einigen Ausführungsformen ist die erfindungsgemäße Steuer- oder Regelvorrichtung programmiert, die Blutbehandlungsvorrichtung derart zu steuern oder zu regeln, dass sichergestellt ist, dass der Effluentbeutel nicht über ein vorbestimmtes Maß oder Volumen befüllt wird, insbesondere, dass dieser nicht platzt und Verunreinigungen verursacht.

Beispielsweise kann mittels einer Waage das Füllvolumen oder mittels eines Sensors der Füllstand des Effluentbeutels überwacht werden. Wird z. B. ein vorbestimmtes Gewicht oder eine vorbestimmte Pegelhöhe erreicht oder überschritten, so kann die Steuer- oder Regelvorrichtung, insbesondere automatisch, veranlassen, dass die Bilanzierung und/oder wenigstens eine der vorgenannten Pumpen angehalten und der Effluentbeutel, z. B. mittels der Effluentbeutelentleerungsvorrichtung automatisch entleert wird. Die Steuer- und Regelvorrichtung kann ferner programmiert sein, ein anschließendes Fortsetzen der Blutbehandlungssitzung, der Bilanzierung, der Pumpentätigkeit usw., z. B. ebenfalls automatisch, zu veranlassen.

Hierbei soll die Steuer- oder Regelvorrichtung optional kontrollieren und/oder optional steuernd oder regelnd derart eingreifen, dass der Effluentbeutel nicht über ein vorbestimmtes Maß befüllt wird, beispielsweise wenn ein Entleerungsintervall übersprungen wurde, was insbesondere auch manuell veranlasst werden kann. In diesen Ausführungsformen kann die festgestellte Pegelhöhe im Effluentbeutel und/oder das festgestellte Gewicht des Effluentbeutels genutzt werden, um eine Entleerung zu starten. Eine entsprechende Überwachung von Pegel und/oder Gewicht durch die Blutbehandlungsvorrichtung kann vorgesehen sein. Wenn ein Betriebszustand auftreten sollte, in dem die Entleerung mittels Timings (d. h. unter Betrachtung der Unterbrechungszeitpunkte) nicht ausreichend ist und z. B. die Waage oder der Pegelsensor erkennt, dass der Effluentbeutel voll oder zu voll ist, so kann automatisch ein Unterbrechungsintervall ausgelöst oder getriggert werden. In diesem Fall kann beispielsweise der Benutzer zu einem manuellen Starten des Entleerungsvorgangs aufgefordert werden.

Das Kontrollieren, Steuern oder Regeln kann beispielsweise vorsehen, auf Daten von mit der Blutbehandlungsvorrichtung oder der Effluentbeutelentleerungsvorrichtung in Signalverbindung stehenden Pumpen, Waagen oder Sensoren zurückzugreifen, um eine Füllhöhe oder -gewicht des Effluentbeutels zu ermitteln und/oder zu überwachen. Das Überwachen kann ein Vergleichen mit Sollwerten und/oder Maximalwerten beinhalten.

In manchen Ausführungsformen ist die Blutbehandlungsvorrichtung als Hämodialysevorrichtung, Hämofiltrationsvorrichtung oder Hämodiafiltrationsvorrichtung, insbesondere als eine Vorrichtung für die akute, die chronische Nierenersatztherapie oder für die kontinuierliche Nierenersatztherapie (CRRT = continuous renal replacement therapy) ausgestaltet.

In einigen Ausführungsformen ist die Steuervorrichtung der erfindungsgemäßen Effluentbeutelentleerungsvorrichtung programmiert, um mittels des Pumpenabschnitts über eine vorbestimmte Entleerungszeitdauer Effluent über die (oder mittels der) Effluentablaufleitung aus dem Effluentbeutel heraus zu fördern.

In manchen Ausführungsformen steht die Steuervorrichtung der Effluentbeutelentleerungsvorrichtung in Signalverbindung mit einem Signalgeber oder einer Uhr. Die Uhr kann insbesondere eine Uhr der Effluentbeutelentleerungsvorrichtung sein, sie kann auch in der Effluentbeutelentleerungsvorrichtung sein. Die Uhr kann eine externe Uhr sein, insbesondere die optionale Uhr der Blutbehandlungsvorrichtung, eine Netzwerkuhr, eine Stationsuhr oder eine Atomuhr. Die Steuervorrichtung ist hierbei konfiguriert, um bei Erhalten eines vorbestimmten Signals vom Signalgeber oder bei Erreichen einer vorbestimmten Uhrzeit auf der Uhr (oder ggf. nach Erhalten eines entsprechenden Signals von der Uhr) den Zeitpunkt des Erhalts des Signals oder das Erreichen der vorbestimmten Uhrzeit als Entleerungszeitpunkt zu definieren oder in Abhängigkeit hiervon, z. B. in einer zeitlichen Abhängigkeit, festzusetzen.

In einigen Ausführungsformen des erfindungsgemäßen Systems liegen Blutbehandlungsvorrichtung und Effluentbeutelentleerungsvorrichtung getrennt voneinander vor, insbesondere während der Behandlung des Patienten, die mittels der Blutbehandlungsvorrichtung erfolgt und/oder während des Leerens des Effluentbeutels, von einer Fluidverbindung zwischen Effluentbeutel und Blutbehandlungsvorrichtung und/oder zwischen Effluentbeutel und Effluentbeutelentleerungsvorrichtung abgesehen.

In manchen Ausführungsformen des Systems sind die Steuer- oder Regelvorrichtung der Blutbehandlungsvorrichtung und die Steuervorrichtung der Effluentbeutelentleerungsvorrichtung jeweils derart programmiert, dass wenigstens ein Unterbrechungszeitpunkt und wenigstens ein Entleerungszeitpunkt gleichzeitig oder um eine vorbestimmte Zeitdauer oder Karenzzeit versetzt zueinander eintreten, insbesondere miteinander in vorbestimmter zeitlicher Beziehung stehen.

Das System kann derart konfiguriert sein, dass die Unterbrechungsintervalle der Blutbehandlungsvorrichtung mit den Entleerungsintervallen der Effluentbeutelentleerungsvorrichtung in Zusammenhang, z. B. einem zeitlichen Zusammenhang, stehen, um das Entleeren eines Effluentbeutels, d. h. ein Entleerungsintervall, der Effluentbeutelentleerungsvorrichtung genau dann zu triggern, wenn an der Blutbehandlungsvorrichtung ein Unterbrechungsintervall vorliegt.

In einigen Ausführungsformen des Systems sind die Steuer- oder Regelvorrichtung der Blutbehandlungsvorrichtung und die Steuervorrichtung der Effluentbeutelentleerungsvorrichtung jeweils derart programmiert, dass wenigstens eine Unterbrechungszeitdauer (oder ein Unterbrechungsintervall) und eine Entleerungszeitdauer (oder ein Entleerungsintervall) einander überlappen, insbesondere zu zumindest 50 %, 60 %, 70 %, 80 % oder 90 % der Dauer einer der beiden.

In manchen Ausführungsformen des Systems sind die Uhr, mit welcher die Steuer- oder Regelvorrichtung der Blutbehandlungsvorrichtung in Signalverbindung steht, und die Uhr, mit welcher die Steuervorrichtung der Effluentbeutelentleerungsvorrichtung in Signalverbindung steht, identisch oder synchronisiert, d. h. es sind entweder ein und dieselbe Uhr oder zwei Uhren, welche die identische Uhrzeit oder zumindest vergleichbare Uhrzeiten anzeigen, angeben oder ausgeben. Alternativ kann dem System bekannt sein, wie groß die in aller Regel konstante Abweichung beider Uhrzeiten voneinander ist. Insbesondere orientieren sich die beiden Uhren an derselben Zeitzone (z. B. Mitteleuropäische Zeit (MEZ)).

In manchen Ausführungsformen treten der vorbestimmte Unterbrechungszeitpunkt und/oder der vorbestimmte Entleerungszeitpunkt jeweils zu einer vorbestimmten Uhrzeit ein, z. B. jeweils zur vollen Stunde (beispielsweise 15:00 Uhr, 16:00 Uhr usw.), jede halbe Stunde (beispielsweise 15:00 Uhr, 15:30 Uhr, 16:00 Uhr, 16:30 Uhr usw.), usw.

In einigen Ausführungsformen treten der vorbestimmte Unterbrechungszeitpunkt oder der vorbestimmte Entleerungszeitpunkt eine vorbestimmte Anzahl an Zeiteinheiten (z. B. Minuten, halbe Stunden, Viertelstunden oder Teilen hiervon) nach einem definierten Ereignis ein. So kann z. B. ein beliebiger Zeitpunkt vom Hersteller oder vom Benutzer fest oder veränderbar vorbestimmt sein und z. B. 30 Minuten z. B. nach dem Start der Behandlungssitzung, nach dem Drücken der Starttaste, nach erstmaligem Anlaufen oder Anhalten der Bilanzierung, der Ultrafiltrationspumpe oder einer anderen Pumpe oder, nach einem, diesem Unterbrechungs- oder Entleerungszeitpunkt vorangegangenem ersten, zweiten, dritten Unterbrechungs- oder Entleerungszeitpunkt **usw.** liegen.

In manchen Ausführungsformen beginnt eine Unterbrechungszeitdauer (oder ein Unterbrechungsintervall) mit einem Unterbrechungszeitpunkt und/oder beginnt eine Entleerungszeitdauer (oder ein Entleerungsintervall) mit einem Entleerungszeitpunkt.

In manchen Ausführungsformen ist der Pumpenabschnitt der erfindungsgemäßen Effluentbeutelentleerungsvorrichtung disposable, ein Wegwerf- oder Einmalartikel.

In einigen Ausführungsformen weist der Pumpenabschnitt einen Impeller oder Pumpenrotor auf.

In manchen Ausführungsformen steht die Effluentablaufleitung mit dem Pumpenabschnitt in Förderverbindung.

In manchen Ausführungsformen ist der Pumpenabschnitt mit einem optional hiervon lösbaren Pumpenantrieb verbunden oder mit einem solchen verbindbar.

In einigen Ausführungsformen weist der Pumpenabschnitt oder der Pumpenantrieb eine wiederaufladbare Spannungsquelle, z. B. einen Akku, auf.

In einigen Ausführungsformen des erfindungsgemäßen Verfahrens wird das Effluent mittels des Pumpenabschnitts aus dem Effluentbeutel gefördert.

In manchen Ausführungsformen des Verfahrens erfolgt das Öffnen des Absperrelements manuell, in anderen automatisch.

In einigen Ausführungsformen kann der Effluentbeutel ein Behälter jeder Art sein, beispielsweise ein Container mit flexibler Außenhaut wie einer Folie, oder aus Folie bestehen, eine harte Außenhaut wie ein Kanister haben, usw.

Geeignete, optionale Schlauchverbinder oder Verbinderpaare oder Konnektorpaare können vorgesehen sein, um das Verbinden der Effluentablaufleitung mit dem Effluentbeutel und/oder mit dem Ausguss zu erleichtern.

In manchen Ausführungsformen ist der Pumpenabschnitt magnetisch gelagert oder angetrieben und weist insbesondere den Pumpkopf auf. Dieser Pumpkopf ist beispielsweise ausgestaltet als Impellerpumpkopf oder als dessen Rotor. Diese elektrisch isolierte Art der Lagerung oder des Antriebs dient dem Schutz des Patienten vor einem elektrischen Stromschlag.

In einigen Ausführungsformen wird der Pumpenantrieb manuell mit dem Pumpenabschnitt, der den Pumpkopf beinhaltet, verbunden.

In manchen Ausführungsformen ist der Pumpenantrieb optional mit einem Magnetabschnitt ausgestaltet, z. B. ausgestaltet als Spulen mit Eisenkern. Dieser dient zum magnetischen Ankoppeln und/oder magnetischen Antreiben von Bauteilen, insbesondere eines magnetisch angetriebenen Pumpenrotors. Der Antrieb ist vorzugweise kontaktlos.

In einigen Ausführungsformen ist eine Induktionsladestation zum Laden des Akkus des Pumpenantriebs mit umfasst. Die Induktionsladestation besitzt optional eine Steuerelektronik, welche konfiguriert ist, um das Aufliegen des Pumpenantriebs auf der Induktionsladestation zu erkennen und den Akku zu laden.

Die Induktionsladestation kann eine Plattform oder Aufstellebene für den Pumpenantrieb sein oder aufweisen.

Die vorliegende Erfindung betrifft eine Effluentbeutelentleerungsvorrichtung, welche wenigstens einen Pumpenabschnitt mit einem Pumpenrotor aufweist. Der Pumpenabschnitt ist vorgesehen, um funktionell mit einem Pumpenantrieb verbunden oder zusammengebracht zu werden. Der Pumpenabschnitt, und optional auch alle anderen Abschnitte der Effluentbeutelentleerungsvorrichtung, weisen in einigen Ausführungsformen keine Einrichtung(en) zum Verbinden des Pumpenabschnitts mit dem Pumpenantrieb auf, abgesehen von einer optionalen Magnetverbindung und/oder Nutzung der Schwerkraft.

In manchen Ausführungsformen weist der Pumpenantrieb eine Induktionsladespule zum Laden der Spannungsquelle oder des Akkus auf.

In einigen Ausführungsformen weist das Gehäuse des Pumpenantriebs einen Pumpenantriebs-Aufstellabschnitt zum Aufstellen des Pumpenantriebs auf einer Pumpenantriebs-Aufstellfläche auf.

In manchen Ausführungsformen weist das Gehäuse des Pumpenantriebs einen Verbindungsabschnitt zum, insbesondere funktionellen, Verbinden des Pumpenantriebs mit dem Pumpenabschnitt, welcher einen Pumpenrotor beinhaltet, auf. Der Verbindungsabschnitt kann hierbei eine Kavität, eine oder mehrere Öffnungen, eine oder mehrere Sacköffnungen oder dergleichen sein.

In einigen Ausführungsformen liegen der Pumpenantriebs-Aufstellabschnitt und der Verbindungsabschnitt an gegenüberliegenden Enden des Gehäuses des Pumpenantriebs oder sind diesen zugeordnet.

In manchen Ausführungsformen weist der Pumpenantrieb wenigstens eine, insbesondere farbige, Leuchteinrichtung auf. Diese kann insbesondere als Ring, insbesondere als LED oder LED-Farbring, ausgestaltet sein.

Die Leuchteinrichtung kann insbesondere im oder am Gehäuse des Pumpenantriebs positioniert sein.

In einigen Ausführungsformen ist die Steuerelektronik des Pumpenantriebs mit einem Wireless Modul ausgestaltet oder mit einem solchen verbunden.

In manchen Ausführungsformen ist die Steuerelektronik mit wenigstens einem Bewegungssensor ausgestaltet oder verbunden.

In manchen Ausführungsformen ist die Steuerelektronik konfiguriert, die Leuchteinrichtung bei oder nach Erreichen des Zustandes der vollständigen Ladung der Spannungsquelle oder des Akkus auszuschalten.

In manchen Ausführungsformen beträgt die bevorzugte elektrischen Nennleistung zwischen 25 und 40 Watt (W), insbesondere 30 bis 35 W, ganz besonders 32 W.

In einigen Ausführungsformen beträgt die Betriebsspannung des elektrischen Antriebs der Pumpe 24 V.

In manchen Ausführungsformen weist der Pumpenabschnitt eine Einrichtung zum lösbaren Befestigen desselben an einem Abschnitt der Effluentbeutelentleerungsvorrichtung, insbesondere zu seinem lösbaren Befestigen an einem Aufstellabschnitt der Effluentbeutelentleerungsvorrichtung oder am Fuß der Blutbehandlungsvorrichtung, auf.

In einigen Ausführungsformen ist die Einrichtung zum lösbaren Befestigen des Pumpenabschnitts eine Klemmeinrichtung oder eine Rast- oder Clipeinrichtung oder weist eine solche auf.

In einigen Ausführungsformen hat die Einrichtung zum lösbaren Befestigen des Pumpenabschnitts, die optional eine Klemmeinrichtung oder eine Rast- oder Clipeinrichtung ist, einen gebogenen Verlauf.

Der gebogene Verlauf kann die Form eines entlang seiner Längserstreckung gekrümmten Halbkanals aufweisen.

In einigen Ausführungsformen weist der Pumpenantrieb, der Pumpenabschnitt und/oder ein anderer Bestandteil der Pumpe jedenfalls keine mechanische Verbindungseinrichtung zum Verbinden des Pumpenantriebs mit dem Pumpenabschnitt auf, außer der Aufstellfläche des Pumpenabschnitts, auf welche der Pumpenantrieb zum Erzielen der funktionsfähigen Pumpe aufgestellt wird.

Ein hiermit erzielbarer Vorteil kann darin bestehen, dass der Pumpenantrieb zu jeder Zeit (auch im laufenden Pumpvorgang) einfach nach oben abgezogen oder abgehoben werden kann. Dies kann insbesondere einhändig erfolgen, insbesondere, wenn keine mechanischen Verbindungseinrichtungen zuvor geöffnet werden müssen, da letztere in diesen Ausführungsformen nicht vorgesehen sind.

In manchen Ausführungsformen ist der Pumpenabschnitt mit seinem Flüssigkeitseinlass oder Fluideinlass mit einem Schlauchabschnitt verbunden, der vom Effluentbeutel zur Pumpe führt.

In einigen Ausführungsformen ist der Pumpenabschnitt mit seinem Flüssigkeitsauslass oder Fluidauslass mit der Ausgussleitung verbunden, die zu einem Ablass, Ausgussbecken oder Ausguss zum Verwerfen des Effluents führen kann.

In manchen Ausführungsformen ist der Pumpenrotor magnetisch gelagert und/oder magnetisch angetrieben.

Der Magnetabschnitt des Pumpenrotors kann ein Permanentmagnet sein.

In manchen Ausführungsformen beträgt die Speicherkapazität des Akkus des Pumpenantriebs zwischen 800 mAh und 1800 mAh, bevorzugt zwischen 1000 mAh und 1500 mAh, ganz besonders bevorzugt etwa oder genau 1100 mAh.

In manchen Ausführungsformen steht der Fluideinlass des Pumpenabschnitts während der Behandlungssitzung mittels Schlauchs oder Leitung in Fluidverbindung mit dem Effluentbeutel. In anderen Ausführungsformen ist der Fluideinlass des Pumpenabschnitts während der Behandlungssitzung getrennt vom Effluentbeutel.

In einigen Ausführungsformen weist die Effluentablaufleitung wenigstens ein Rückschlagventil, z. B. stromauf des Pumpenantriebs, auf. Das Rückschlagventil kann bei abgekoppeltem Effluentbeutel ein ungewolltes Austreten von Effluent aus der Effluentablaufleitung vorteilhaft verhindern, was der Sauberkeit und Hygiene zugutekommt.

In einigen Ausführungsformen weist die Effluentablaufleitung, die sich stromab des Effluentbeutels anschließt, stromauf und/oder stromab des Pumpenabschnitts der Effluentbeutelentleerungsvorrichtung kein Element auf, das mit einer elektrischen Steuereinrichtung verbunden wäre, etwa in Form eines elektrisch verbundenen Verbinders. Die Pumpe bzw. der Pumpenabschnitt sind optional hiervon ausgenommen; sie können elektrisch verbunden sein. Optional sind aber auch sie nicht elektrisch mit z. B. einer Steuervorrichtung verbunden.

In einigen Ausführungsformen ist die Pumpe dergestalt, beispielsweise als Impellerpumpe, konfiguriert, dass sie einen mit zehn Liter Effluent gefüllten Effluentbeutel zumindest bei maximal hohem Fluss in rund fünf Minuten oder weniger, vorzugsweise in rund zwei Minuten, leer pumpen kann.

In manchen Ausführungsformen umfasst die erfindungsgemäße Effluentbeutelentleerungsvorrichtung eine Beutelaufnahme zum Halten oder Aufnehmen eines Effluentbeutels mit einer Effluentauslassöffnung. Optional weisen der Effluentbeutel oder die Effluentbeutelentleerungsvorrichtung ein Absperrelement zum zeitweisen Verschließen der Effluentauslassöffnung auf.

In manchen Ausführungsformen erfolgt das Unterbrechen durch die erfindungsgemäße Steuer- oder Regelvorrichtung, oder von ihr veranlasst, bei noch angehängtem Effluentbeutel, und vorzugsweise während dieser noch nicht leergepumpt wird.

Manche oder alle erfindungsgemäßen Ausführungsformen können einen, mehrere oder alle der oben und/oder im Folgenden genannten Vorteile aufweisen.

Ein Vorteil der vorliegenden Erfindung kann darin bestehen, dass das Entleeren des Effluentbeutels in den Behandlungsverlauf der extrakorporalen Blutbehandlung eines Patienten so integriert werden kann, dass die Flüssigkeitsbilanzierung während der Blutbehandlung nicht gestört oder verfälscht wird. Auch sind vorteilhafterweise keine Vorrichtungen vorzusehen, die eine fortlaufende und zuverlässige Bilanzierung auch während des Entleerens des Effluentbeutels mit dem Ziel einer unverfälschten Bilanzierung sicherstellen müssen.

Ein weiterer Vorteil kann darin bestehen, dass der Effluentbeutel durch sein Entleeren mittels der erfindungsgemäßen Effluentbeutelentleerungsvorrichtung nicht mehr vom Klinikpersonal zu einem Ausguss, z. B. einem Waschbecken, einer Toilette, einem Gully etc. getragen werden muss. Da volle Effluentbeutel in der Regel um die 10 kg wiegen, wird das Klinikpersonal bei dieser Tätigkeit erheblich entlastet, wenn die mobile, separat verwendbare Effluentbeutelentleerungsvorrichtung z. B. unmittelbar neben der Blutbehandlungsvorrichtung platziert wird und der zu entleerende Effluentbeutel während seines Entleerens an der Blutbehandlungsvorrichtung angeordnet bleiben kann.

Herkömmliche Systeme und Vorrichtungen zum Entleeren des Effluentbeutels sind teils sehr aufwändig und unterliegen außerdem z. T. der Zulassung als medizinisches Gerät. Manche dieser Systeme erfordern z. B. eine aufwendige Maschinenhardware der Dialysemaschine, insbesondere spezielle Wiege- oder Wägesysteme. Nachrüstungen solcher Systeme mit dem Ziel, das Entleeren des Effluentbeutels zu erleichtern, erfordern Veränderungen der Hardware der Blutbehandlungsvorrichtung, die mit hohem Zeit- und Kostenaufwand für die Neuzulassung der veränderten Maschine verbunden sein können. Eine Nachrüstung vorhandener Blutbehandlungsvorrichtungen ist aus diesem Grunde oftmals nicht vertretbar. Durch die vorliegende Erfindung kann nunmehr kostengünstig und einfach ein System zum Entleeren von Effluentbeuteln zur Verfügung gestellt werden, das keiner Zulassung als medizinisches Gerät bedarf und das auch dazu beitragen kann, dass eine erneute Zulassung der Blutbehandlungsvorrichtung, deren Effluentbeutel geleert werden soll, nicht erforderlich ist. Vielmehr können mittels der vorliegenden Erfindung bestehende Blutbehandlungsvorrichtungen auf einfache Weise ergänzt werden, indem man die erfindungsgemäße Effluentbeutelentleerungsvorrichtung in der Nähe der Blutbehandlungsvorrichtung positioniert und zum Entleeren des Effluentbeutels einsetzt. Die Steuer- oder Regelvorrichtung der Blutbehandlungsvorrichtung muss allein auf gelegentliche Unterbrechungen, z. B. der Bilanzierung, oder zum Übermitteln einer Information darüber, wann solche Unterbrechungen stattfinden, programmiert werden oder solche Unterbrechungen der Effluentbeutelentleerungsvorrichtung per Signal mitgeteilt werden. Aus dem Markt bekannte Effluentbeutel können vorteilhaft zusammen mit den erfindungsgemäßen Vorrichtungen auch weiterhin verwendet werden.

Durch die vorliegende Erfindung kann vorteilhafterweise weiter das Risiko umgangen werden, dass beim Ablassen des Inhalts des Effluentbeutels elektrischer Kontakt zwischen der Flüssigkeit in der Effluentablaufleitung oder einer anderen Effluent führenden Leitung und der Erde auftritt, sodass die zulässigen Patientenableitströme im Fehlerfall überschritten würden, da die erfindungsgemäße Effluentbeutelentleerungsvorrichtung selbst an keine Stromversorgung angeschlossen werden muss. Von der Benutzung der akku-betriebenen Pumpe geht keine Gefahr eines Elektrounfalls oder Spannungsschadens für den Patienten aus.

Ein weiterer Vorteil der vorliegenden Erfindung kann darin bestehen, dass durch die regelmäßige bis quasi kontinuierliche Entleerung des Effluentbeutels bislang erforderliche Beutelwechselintervalle für den Effluentbeutel, oder Intervalle, um diesen zu entleeren, zumindest teilweise entfallen können.

Ein weiterer Vorteil der vorliegenden Erfindung kann darin bestehen, dass durch ihre Verwendung Arbeitsabläufe an der Blutbehandlungsvorrichtung optimiert werden können, da das Entleeren des vollen Effluentbeutels z. B. während ohnehin erfolgender Unterbrechungen der Behandlungsflüsse erfolgen kann.

Alle mit dem erfindungsgemäßen Verfahren erzielbaren Vorteile lassen sich ungeschmälert auch mit den erfindungsgemäßen Vorrichtungen erzielen, und umgekehrt.

Im Folgenden wird die vorliegende Erfindung unter Bezugnahme auf die beigefügte Zeichnung rein exemplarisch beschrieben. In ihr bezeichnen selbe Bezugszeichen gleiche oder selbe Komponenten. In den Figuren der Zeichnungen gilt:
- **Fig. 1**: zeigt in vereinfachter Darstellung ein Verfahrensfließbild einer erfindungsgemäßen Blutbehandlungsvorrichtung mit einem extrakorporalen Blutkreislauf und einer erfindungsgemäßen Steuer- oder Regelvorrichtung;
- **Fig. 2**: zeigt eine erfindungsgemäße Effluentbeutelentleerungsvorrichtung in einer ersten Ausführungsform; und
- **Fig. 3**: zeigt ein stark vereinfachtes Diagramm eines Volumenstroms in einer Effluentzulaufleitung einer erfindungsgemäßen Blutbehandlungsvorrichtung über der Zeit (oben) und ein Diagramm des Volumenstroms in der Effluentablaufleitung einer erfindungsgemäßen Effluentbeutelentleerungsvorrichtung über der Zeit (unten).

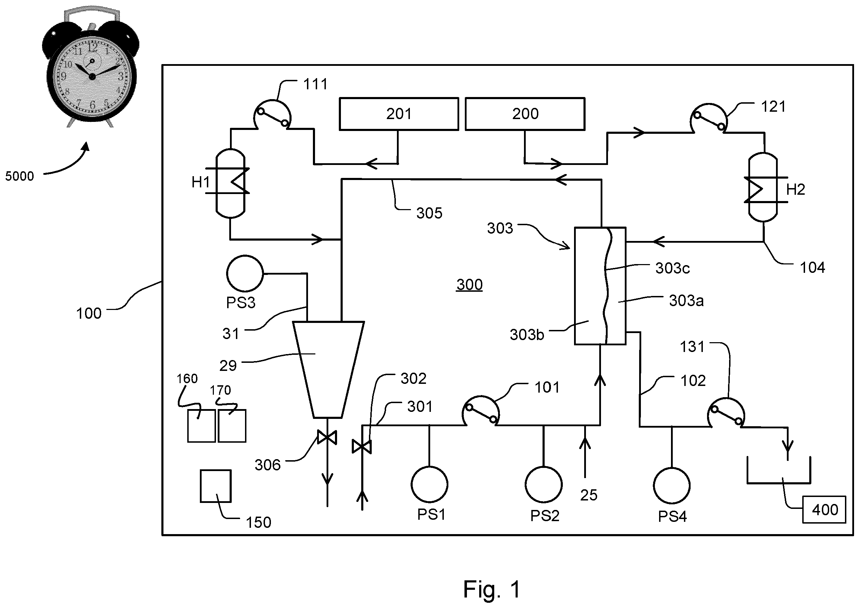

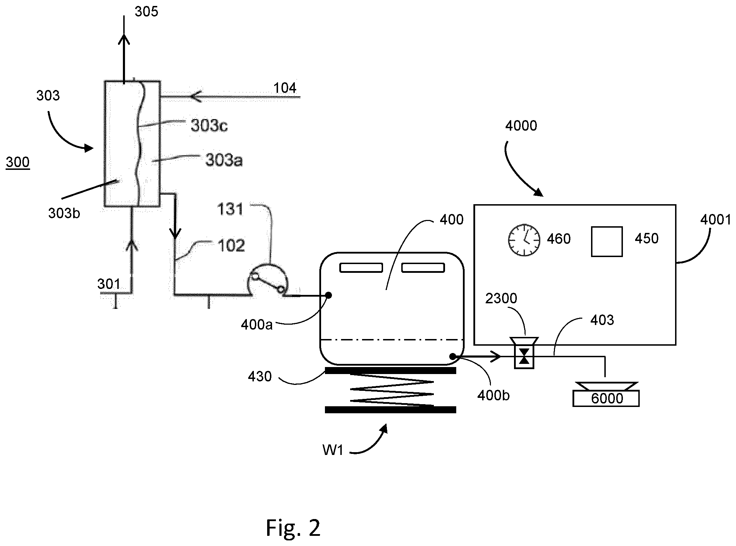

**Fig. 1** zeigt in stark vereinfachter Darstellung ein Verfahrensfließbild einer erfindungsgemäßen Blutbehandlungsvorrichtung 100, optional verbunden mit einem extrakorporalen Blutkreislauf 300 und einem Abflussschlauchsystem zu einem Effluentbeutel 400. Der Effluentbeutel 400 kann Teil der Blutbehandlungsvorrichtung 100 oder Teil der in Fig. 2 gezeigten Effluentbeutelentleerungsvorrichtung 4000 sein.

Der extrakorporale Blutkreislauf 300 weist eine erste Leitung 301, hier in Form eines arteriellen Leitungsabschnitts, auf.

Die erste Leitung 301 steht in Fluidverbindung mit einer Blutbehandlungseinrichtung, hier exemplarisch ein Blutfilter oder Dialysator 303. Der Blutfilter 303 weist eine Dialysierflüssigkeitskammer 303a und eine Blutkammer 303b auf, welche durch eine zumeist semi-permeable Membran 303c voneinander getrennt sind.

Der extrakorporale Blutkreislauf 300 weist ferner wenigstens eine zweite Leitung 305, hier in Form eines venösen Leitungsabschnitts, auf. Sowohl die erste Leitung 301 als auch die zweite Leitung 305 können zu ihrer Verbindung mit dem Gefäßsystem des nicht dargestellten Patienten dienen.

Die erste Leitung 301 ist optional mit einer (ersten) Schlauchklemme 302 zum Sperren oder Schließen der Leitung 301 verbunden. Die zweite Leitung 305 ist optional mit einer (zweiten) Schlauchklemme 306 zum Sperren oder Schließen der Leitung 305 verbunden.

Die in Fig. 1 nur durch einige ihrer Einrichtungen und schematisch repräsentierte Blutbehandlungsvorrichtung 100 weist eine Blutpumpe 101 auf. Die Blutpumpe 101 fördert während der Behandlung des Patienten Blut durch Abschnitte des extrakorporalen Blutkreislaufs 300 und in Richtung zum Blutfilter oder Dialysator 303, wie die kleinen Pfeilspitzen, welche in jeder der Figuren allgemein die Strömungsrichtung angeben, zeigen.

Mittels einer Pumpe für Dialysierflüssigkeit 121, die als Rollenpumpe oder als anderweitig okkludierende Pumpe ausgestaltet sein kann, wird frische Dialysierflüssigkeit aus einer Quelle 200 entlang der Dialysierflüssigkeitszulaufleitung 104 in die Dialysierflüssigkeitskammer 303a gepumpt. Die Dialysierflüssigkeit verlässt die Dialysierflüssigkeitskammer 303a als Dialysat, ggf. angereichert durch Filtrat, in Richtung des Effluentbeutels 400 und wird hierin als Effluent bezeichnet.

Die Quelle 200 kann beispielsweise ein Beutel oder ein Container sein. Die Quelle 200 kann ferner eine Fluidleitung sein, aus der online und/oder kontinuierlich erzeugte oder gemischte Flüssigkeit bereitgestellt wird, z. B. ein Hydraulikausgang oder -anschluss der Blutbehandlungsvorrichtung 100.

Eine weitere Quelle 201 mit Substituat kann optional vorgesehen sein. Sie kann der Quelle 200 entsprechen oder eine eigene Quelle sein.

Eine nur angedeutete Steuer- oder Regelvorrichtung 150 kann dazu konfiguriert sein, die Blutbehandlungssitzung zu regeln oder zu steuern.

Die Steuer- oder Regelvorrichtung 150 kann dazu vorgesehen sein mittels einer nur angedeuteten Uhr 160, optional mittels eines Signalgebers 170, ein Anhalten einer Bilanzierung der Blutbehandlungsvorrichtung 100 zu veranlassen, z. B. zu vorgegebenen Uhrzeiten oder nach vorgegebenen Zeitintervallen.

Alternativ kann die Uhr 160 dazu vorgesehen sein, mit einer externen Uhr 5000, beispielsweise einer Netzwerkuhr, einer Stationsuhr oder einer Atomuhr, in Signalverbindung stehen, mit einer solchen Uhr synchronisiert zu werden oder mit ihr identisch zu sein.

Die Uhr 160 und/oder der Signalgeber 170 können weiter optional dazu vorgesehen sein, mit einer Uhr der Effluentbeutelentleerungsvorrichtung 4000, wie zu Fig. 2 beschrieben, in Signalverbindung zu stehen, z. B. um mit dieser synchronisiert zu werden.

Alternativ oder ergänzend kann der Signalgeber 170 in Signalverbindung mit der Steuervorrichtung 450 der Effluentbeutelentleerungsvorrichtung 4000 stehen, um diese zum Veranlassen bzw. Triggern eines Entleerungsintervalls mittels des Pumpenabschnitts 2300, zu veranlassen (siehe Fig. 2).

Rechts unten ist in Fig. 1 angedeutet, wo der Effluentbeutel 400 fluidisch, formschlüssig und/oder kraftschlüssig optional mit der Blutbehandlungsvorrichtung 100 verbunden ist. Eine mit dem Effluentbeutel 400 optional nur fluidisch verbundene Effluentbeutelentleerungsvorrichtung 4000 sowie deren Komponenten sind erst in Fig. 2 gezeigt.

Neben der vorgenannten Blutpumpe 101 weist die in Fig. 1 gezeigte Anordnung ferner rein optional eine Reihe weiterer, jeweils optionaler Pumpen, nämlich die Pumpe 111 für Substituat, die Pumpe 121 für Dialysierflüssigkeit und die Pumpe 131 für das Effluent auf.

Die Pumpe 121 ist vorgesehen, um Dialysierflüssigkeit aus einer Quelle 200, beispielsweise einem Beutel, heraus und über eine optional vorhandene Beutelheizung H2 mit einem Heizbeutel dem Blutfilter 303 mittels der Dialysierflüssigkeitszulaufleitung 104 zuzuführen.

Die auf diese Weise zugeführte Dialysierflüssigkeit tritt über eine Dialysatablaufleitung 102, unterstützt durch die optionale Pumpe 131, wieder aus dem Blutfilter 303 aus und kann verworfen werden.

Stromauf der Blutpumpe 101 ist ein optionaler arterieller Sensor PS1 vorgesehen. Während einer Behandlung des Patienten misst er den Druck in der arteriellen Leitung.

Stromab der Blutpumpe 101, jedoch stromauf des Blutfilters 303 und, falls vorgesehen, stromaufwärts einer Zugabestelle 25 für Heparin, ist ein weiterer, optionaler Drucksensor PS2 vorgesehen. Er misst den Druck stromauf des Blutfilters 303 ("prä-Hämofilter").

Ein wiederum weiterer Drucksensor kann als PS4 stromab des Blutfilters 303, jedoch vorzugsweise stromauf der Pumpe 131, in der Dialysatablaufleitung 102 zum Messen des Filtratdrucks des Blutfilters 303 vorgesehen sein.

Blut, das den Blutfilter 303 verlässt, durchströmt eine optionale venöse Blutkammer 29, welche eine Entlüftungseinrichtung 31 und/oder einen weiteren Drucksensor PS3 aufweisen kann.

Die in Fig. 1 gezeigte Steuer- oder Regelvorrichtung 150 kann mit jeder der hierin genannten Komponenten - jedenfalls oder insbesondere mit der Blutpumpe 101 - in kabelgebundener oder kabelloser Signalverbindung zur Steuerung oder Regelung der Blutbehandlungsvorrichtung 100 stehen.

Die optionale Pumpe 111 ist vorgesehen, um Substituat aus der optionalen Quelle 201, beispielsweise einem Beutel, heraus und über eine optional vorhandene Beutelheizung H1 mit einem Heizbeutel der zweiten Leitung 305 zuzuführen.

**Fig. 2** zeigt eine erfindungsgemäße Effluentbeutelentleerungsvorrichtung 4000 in einer ersten Ausführungsform.

Die Fig. 2 zeigt zudem auf der linken Seite einen Ausschnitt des extrakorporalen Blutkreislaufs 300 einer Blutbehandlungsvorrichtung 100 aus Fig. 1, nämlich den arteriellen Leitungsabschnitt 301, der, bezogen auf die Fig. 2, von unten in die Blutkammer 303b des Dialysators 303 führt. Auf der gegenüberliegenden Seite des Dialysators 303 (in der Fig. 2 oben) führt der venöse Leitungsabschnitt 305 wieder aus der Blutkammer 303b heraus. Von der Blutkammer 303b durch die semi-permeable Membran 303c getrennt liegt die Dialysierflüssigkeitskammer 303a, in welche über die Dialysierflüssigkeitszulaufleitung 104 frische Dialysierflüssigkeit in den Dialysator 303 gelangt. Die Dialysierflüssigkeit wird stromab des Dialysators 303, von nun an als Filtrat oder Effluent bezeichnet, mittels der Pumpe 131 über die Dialysierablaufleitung bzw. Effluentzulaufleitung 102 (da sie dem Effluentbeutel 400 Effluent zuführt) aus der Dialysierflüssigkeitskammer 303a in Richtung Effluentbeutel 400 geführt und dort durch eine Effluenteinlassöffnung 400a in ein Inneres des Effluentbeutels 400 geführt. Der Effluentbeutel 400 ist hier exemplarisch auf oder in einer Beutelaufnahme 430 angeordnet, welche wiederum mit einer Waage W1 bzw. mit einer anderen Wägeeinrichtung verbunden und Teil der Blutbehandlungsvorrichtung 100 oder der Effluentbeutelentleerungsvorrichtung 4000 sein kann.

Eine exemplarische Füllhöhe oder ein exemplarischer Flüssigkeitsspiegel im Innern des Effluentbeutels 400 ist mit einer Punkt-Strich-Linie markiert.

Während eines Unterbrechungsintervalls, während welchem die Bilanzierung der Blutbehandlungsvorrichtung 100 und damit z. B. auch die Pumpe 131 angehalten ist, wird der Pumpenabschnitt 2300 mittels einer Steuervorrichtung 450 aktiviert. Er saugt daraufhin das im Effluentbeutel 400 befindliche Effluent oder Teile hiervon durch die Effluentauslassöffnung 400b, die in Fig. 2 optional rechts unten am Effluentbeutel 400 angeordnet ist, an und pumpt Effluent entlang einer Effluentablaufleitung 403, z. B. in einen Ausguss 6000.

Zum Bestimmen des Beginns der Pumptätigkeit, also zum Bestimmen, wann ein Entleerungszeitpunkt vorliegt, kann in der Effluentbeutelentleerungsvorrichtung 4000 eine Uhr 460 angeordnet sein. Die Effluentbeutelentleerungsvorrichtung 4000 kann optional mit der Uhr 160 der Blutbehandlungsvorrichtung 100, dem Signalgeber 170 oder einer externen Uhr 5000 (siehe Fig. 1) in Signalverbindung stehen.

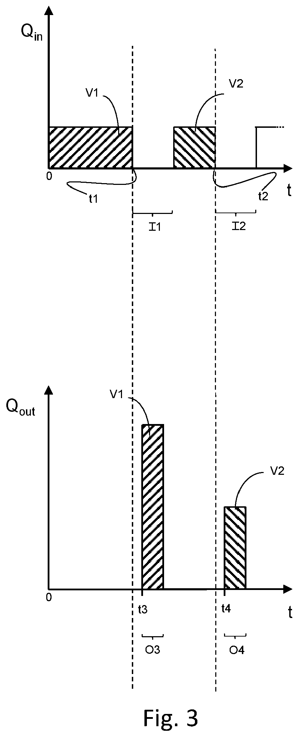

Die Steuervorrichtung 450 der Effluentbeutelentleerungsvorrichtung 4000 kann programmiert sein, um den Entleerungsvorgang zu einem vorgegebenen Zeitpunkt und genau für einen Zeitraum vorbestimmter Dauer, dem Entleerungszeitraum, zu starten und beim Erreichen des Endes dieses Zeitraums vorzugsweise auch zu beenden. Falls möglich liegt dieser Entleerungszeitraum vorzugsweise in einem Unterbrechungsintervall, in dem die Blutbehandlungsvorrichtung 100 steht (Unterbrechungszeitdauer) oder überlappt sich mit diesem. Vorzugsweise wird in diesem Entleerungszeitraum der Effluentbeutel 400 vorzugsweise jeweils vollständig entleert (siehe auch Fig. 3).

Der Begriff "die Blutbehandlungsvorrichtung 100 steht" bedeutet in dieser Ausführungsform, dass zu diesem Zeitpunkt wenigstens eine der Pumpen 111, 121 und 101 (siehe Fig. 1) nicht fördert und/oder die Bilanzierung angehalten ist.

Wie in Fig. 2 angedeutet, können beliebige zwei oder mehr der hierin genannten Komponenten der Effluentbeutelentleerungsvorrichtung 4000 ganz oder teilweise optional in oder an einem gemeinsamen Gehäuse 4001 vorliegen.

Fig. 1 und Fig. 2 zeigen gemeinsam eine mögliche Ausführungsform des erfindungsgemäßen Systems.

**Fig. 3** zeigt in ihrem oberen Diagramm einen exemplarischen, mittels der Pumpe 131 in der Effluentzulaufleitung 102 erzeugten Volumenstrom Qᵢₙ über der Zeit t.

Mittels der Pumpe 131 wird vom Zeitpunkt t=0 bis zum Zeitpunkt t=t1 ein Volumen V1 in den Effluentbeutel 400 hinein gefördert, vom Zeitpunkt t=t1 bis zum Zeitpunkt t=t2 ein Volumen V2. Diese Volumina V1, V2 sind im oberen Diagramm der Fig. 3 jeweils schraffiert dargestellt.

Dem oberen Diagramm kann weiter entnommen werden, dass die Pumpe 131 zu vorbestimmten Zeitpunkten t1 und t2 angehalten wird, und jeweils für die vorbestimmte Länge oder Dauer eines Intervalls I1 bzw. I2 (Unterbrechungsintervalle sind mit I wie *interruption* bezeichnet) nicht fördert.

I1, I2 sind hier jeweils als Unterbrechungszeitdauer zu verstehen, und t1 und t2 als Unterbrechungszeitpunkte, zu welchen I1, I2 jeweils beginnen.

Fig. 3 zeigt in ihrem unteren Diagramm einen Volumenstrom Qₒᵤₜ, mit dem der Pumpenabschnitt 2300 der Effluentablaufleitung 403 der Effluentbeutelentleerungsvorrichtung 4000 über der Zeit t, Effluent aus dem Effluentbeutel 400 heraus fördert.

Dem unteren Diagramm kann entnommen werden, dass der Pumpenabschnitt 2300 zu vorbestimmten Zeitpunkten t3 und t4 aktiviert wird und für die Länge jeweils eines vorbestimmten Intervalls O3 bzw. O4 (Entleerungsintervalle sind hierin mit O wie out bezeichnet) fördert.

O3, O4 sind hier jeweils als Entleerungszeitdauer zu verstehen, und t3 und t4 als Entleerungszeitpunkte, zu welchen O3, O4 jeweils beginnen.

Dem unteren Diagramm ist weiter zu entnehmen, dass der dritte Zeitpunkt t3 um eine optionale Karenzzeit zeitverzögert hinter dem ersten Zeitpunkt t1 und der vierte Zeitpunkt t4 um eine optionale Karenzzeit hinter dem zweiten Zeitpunkt t2 liegen kann (für t1 und t2 siehe jeweils das obere Diagramm). Diese Zeitverzögerungen können jeweils gleich lang sein, müssen aber nicht. Außerdem liegen die Intervalle O3, O4 vorzugsweise innerhalb der Intervalle I1, I2 bzw. überlappen sich mit diesen.

Bevorzugterweise werden innerhalb der Intervalle O3, O4 jeweils dieselben Volumina V1 bzw. V2 mittels des Pumpenabschnitts 2300 aus dem Effluentbeutel 400 herausgefördert, wie sie zuvor vor den Intervallen I1, I2 (im oberen Diagramm) mittels der Pumpe 131 in den Effluentbeutel 400 hineingefördert worden sind. Diese Volumina V1, V2 sind im unteren Diagramm ebenfalls schraffiert dargestellt.

### Bezugszeichenliste

- 25: Zugabestelle für Heparin (optional)
- 29: venöse Blutkammer (optional)
- 31: Entlüftungseinrichtung

- 100: Blutbehandlungsvorrichtung
- 101: Blutpumpe
- 102: Dialysatablaufleitung, Effluentzulaufleitung
- 104: Dialysierflüssigkeitszulaufleitung
- 111: Pumpe für Substituat
- 121: Pumpe für Dialysierflüssigkeit
- 131: Pumpe für Dialysat oder Effluent in Effluentzulaufleitung
- 150: Steuer- oder Regelvorrichtung
- 160: Uhr
- 170: Signalgeber
- 200: Quelle mit Dialysierflüssigkeit
- 201: Quelle mit Substituat, optional

- 300: extrakorporaler Blutkreislauf
- 301: erste Leitung (arterieller Leitungsabschnitt)
- 302: (erste) Schlauchklemme
- 303: Blutfilter oder Dialysator
- 303a: Dialysierflüssigkeitskammer
- 303b: Blutkammer
- 303c: semi-permeable Membran
- 305: zweite Leitung (venöser Leitungsabschnitt)
- 306: (zweite) Schlauchklemme

- 400: Effluentbeutel
- 400a: Effluenteinlassöffnung;
- 400b: Effluentauslassöffnung
- 403: Effluentablaufleitung, Ausgussleitung

- 430: Beutelaufnahme
- 450: Steuervorrichtung
- 460: Uhr

- 2300: Pumpenabschnitt

- 4000: Effluentbeutelentleerungsvorrichtung
- 4001: Gehäuse
- 5000: Uhr
- 6000: Ausguss; Abfluss; Ablauf

- H1: Beutelheizung mit Beutel (Substituat)
- H2: Beutelheizung mit Beutel (Dialysierflüssigkeit)

- I1, I2: Zeitintervall; Unterbrechungsintervall
- O3, O4: Zeitintervall; Entleerungsintervall

- PS1, PS2: arterieller Drucksensor (optional)
- PS3: Drucksensor (optional)
- PS4: Drucksensor zum Messen des Filtratdrucks

- t1: erster Zeitpunkt; Unterbrechungszeitpunkt
- t2: zweiter Zeitpunkt; Unterbrechungszeitpunkt
- t3: dritter Zeitpunkt; Entleerungszeitpunkt
- t4: vierter Zeitpunkt; Entleerungszeitpunkt

- V1: erstes Fördervolumen
- V2: zweites Fördervolumen

- W1: Waage

## Patentansprüche

1. Steuer- oder Regelvorrichtung (150), programmiert zum Steuern oder Regeln einer
Blutbehandlungsvorrichtung (100) während einer mittels eines extrakorporalen Blutschlauchsatzes (300) und der Blutbehandlungsvorrichtung (100) unter Bilanzierung von Flüssigkeitsströmen, Fördern mittels einer Filtratpumpe (131), Fördern mittels einer Substituatpumpe (111) und/oder Fördern mittels einer Dialysierflüssigkeitspumpe (121) während einer Behandlungssitzung durchgeführten Behandlung des Bluts eines Patienten, **dadurch gekennzeichnet, dass** die Steuer- oder Regelvorrichtung (150) weiter programmiert ist zum
- Unterbrechen der Bilanzierung, und/oder
- Unterbrechen des Calciumlösungsflusses oder der Fördertätigkeit der Calciumpumpe, des Citratlösungsflusses oder der Fördertätigkeit der Citratpumpe, des Heparinlösungsflusses oder der Fördertätigkeit der Heparinpumpe, des Filtratflusses oder der Fördertätigkeit der Filtratpumpe (131), des Substituatflusses oder der Fördertätigkeit der Substituatpumpe (111) und/oder des Dialysierflüssigkeitsflusses oder der Fördertätigkeit der Dialysierflüssigkeitspumpe (121),
zu einem oder zu mehreren vorbestimmten Unterbrechungszeitpunkten (t1, t2), die innerhalb der Dauer der Behandlungssitzung liegen.

2. Steuer- oder Regelvorrichtung (150) nach Anspruch 1, wobei das Unterbrechen jeweils für eine vorbestimmte Unterbrechungszeitdauer (I1, I2) erfolgt, beginnend mit dem zugehörigen Unterbrechungszeitpunkt (t1, t2).

3. Steuer- oder Regelvorrichtung (150) nach Anspruch 1 oder Anspruch 2, wobei bei Eintreten des vorbestimmten Unterbrechungszeitpunkts (t1, t2) ein Hinweis oder Alarm an den Benutzer ergehen kann, der ihn zum manuellen Leeren eines Effluentbeutels (400) auffordert, der während der Blutbehandlungssitzung zum Aufnehmen von während der Behandlungssitzung anfallenden Effluents mit der Blutbehandlungsvorrichtung (100) in Fluidverbindung steht.

4. Steuer- oder Regelvorrichtung (150) nach einem der vorangegangenen Ansprüche, in Signalverbindung mit einem Signalgeber (170) oder einer Uhr der Steuer- oder Regelvorrichtung (150) oder einer externen Uhr (5000), insbesondere einer Uhr (160) der Blutbehandlungsvorrichtung (100), einer Netzwerkuhr, einer Stationsuhr oder einer Atomuhr, und wobei die Steuer- oder Regelvorrichtung (150) konfiguriert ist, um bei Erhalten eines vorbestimmten Signals vom Signalgeber (170) oder bei Erreichen einer vorbestimmten Uhrzeit auf der Uhr (160, 5000) den Zeitpunkt des Erhaltens des Signals oder das Erreichen der vorbestimmten Uhrzeit als Unterbrechungszeitpunkt (t1, t2) zu definieren.

5. Steuer- oder Regelvorrichtung (150) nach einem der vorangegangen Ansprüche, welche programmiert ist, zu überwachen, dass der Effluentbeutel (400) nicht über ein vorbestimmtes Maß oder Volumen hinaus befüllt wird.

6. Blutbehandlungsvorrichtung (100), mit einer Steuer- oder Regelvorrichtung (150) gemäß einem der Ansprüche 1 bis 5.

7. Blutbehandlungsvorrichtung (100) nach Anspruch 6, ausgestaltet als Dialysevorrichtung, Hämodialysevorrichtung, Hämofiltrationsvorrichtung oder Hämodiafiltrationsvorrichtung, insbesondere als eine Vorrichtung für die chronische Nierenersatztherapie oder für die kontinuierliche Nierenersatztherapie (CRRT = continuous renal replacement therapy).

8. Effluentbeutelentleerungsvorrichtung (4000), mit
- einer Effluentablaufleitung (403) zum Leiten von Effluent aus einem Effluentbeutel (400) heraus;
- einem Pumpenabschnitt (2300) zum Fördern von Effluent aus dem Effluentbeutel (400) heraus;
**gekennzeichnet durch**
eine Steuervorrichtung (450), programmiert, um den Pumpenabschnitt (2300) zum Fördern von Effluent aus dem Effluentbeutel (400) heraus zu veranlassen, wenn ein vorbestimmter Entleerungszeitpunkt (t3, t4) eintritt.

9. Effluentbeutelentleerungsvorrichtung (4000) nach Anspruch 8, wobei die Steuervorrichtung (450) programmiert ist, um mittels des Pumpenabschnitts (2300) während einer vorbestimmten Entleerungszeitdauer (O3, O4) Effluent über die Effluentablaufleitung (403) aus dem Effluentbeutel (400) heraus zu fördern.

10. Effluentbeutelentleerungsvorrichtung (4000) nach Anspruch 8 oder Anspruch 9, wobei die Steuervorrichtung (450) in Signalverbindung mit einem Signalgeber (170) oder einer Uhr steht, insbesondere einer Uhr (160) der Blutbehandlungsvorrichtung (100), einer Uhr (460) der Effluentbeutelentleerungsvorrichtung (4000) oder einer externen Uhr (5000), insbesondere einer Netzwerkuhr, einer Stationsuhr oder einer Atomuhr, und wobei die Steuer- oder Regelvorrichtung (150) konfiguriert ist, um bei Erhalt eines vorbestimmten Signals vom Signalgeber (170) oder bei Erreichen einer vorbestimmten Uhrzeit auf der Uhr den Zeitpunkt des Erhalts des Signals oder das Erreichen als Entleerungszeitpunkt (t3, t4) zu definieren oder in Abhängigkeit hiervon festzusetzen.

11. System umfassend eine Blutbehandlungsvorrichtung (100) gemäß einem der Ansprüche 6 bis 7 und eine Effluentbeutelentleerungsvorrichtung (4000) gemäß einem der Ansprüche 8 bis 10.

12. System nach Anspruch 11, wobei die Blutbehandlungsvorrichtung (100) und die Effluentbeutelentleerungsvorrichtung (4000) getrennt voneinander vorliegen.

13. System nach Anspruch 11 oder 12, wobei die Steuer- oder Regelvorrichtung (150) der Blutbehandlungsvorrichtung (100) und die Steuervorrichtung (450) der Effluentbeutelentleerungsvorrichtung (4000) jeweils derart programmiert sind, dass wenigstens ein Unterbrechungszeitpunkt (t1, t2) und wenigstens ein Entleerungszeitpunkt (t3, t4) gleichzeitig oder um eine vorbestimmte Zeitdauer versetzt zueinander eintreten.

14. System nach einem der Ansprüche 11 bis 13, wobei die Steuer- oder Regelvorrichtung (150) der Blutbehandlungsvorrichtung (100) und die Steuervorrichtung (450) der Effluentbeutelentleerungsvorrichtung (4000) jeweils derart programmiert sind, dass wenigstens eine Unterbrechungszeitdauer (I1, I2) und eine Entleerungszeitdauer (O3, O4) einander überlappen, insbesondere zu zumindest 50 %, 60 %, 70 %, 80 % oder 90 % der Dauer einer der beiden.

15. System nach einer der Ansprüche 11 bis 14, wobei der Signalgeber (170) oder die Uhr, mit welcher die Steuer- und Regelvorrichtung (150) der Blutbehandlungsvorrichtung (100) in Signalverbindung steht, und der Signalgeber (170) oder die Uhr, mit welcher die Steuervorrichtung (450) der Effluentbeutelentleerungsvorrichtung (4000) in Signalverbindung steht, identisch oder synchronisiert sind.

## Claims

1. A control or regulating device (150) programmed to control or regulate a blood treatment apparatus (100) during a treatment session treating a patient's blood carried out by means of an extracorporeal blood tubing set (300) and the blood treatment apparatus (100), whilst balancing liquid flows, conveying using a filtrate pump (131), conveying using a substitute pump (111) and/or conveying using a dialysis liquid pump (121), **characterized in that** the control or regulating device (150) is further programmed to:
- interrupt the balancing, and/or
- interrupt the calcium solution flow or the conveying action of the calcium pump, the citrate solution flow or the conveying action of the citrate pump, the heparin solution flow or the conveying action of the heparin pump, the filtrate flow or the conveying action of the filtrate pump (131), the substitute flow or conveying action of the substitute pump (111) and/or the dialysis liquid flow or the conveying action of the dialysis liquid pump (121),
at one or more predetermined interruption time points (t1, t2) which fall within the duration of the treatment session.

2. The control or regulating device (150) according to claim 1, wherein the interruption takes place in each case for a predetermined interruption period (I1, I2), beginning with the corresponding interruption time point (t1, t2).

3. The control or regulating device (150) according to claim 1 or claim 2, wherein, upon reaching the predetermined interruption time point (t1, t2), a notification or alarm may be issued to the user, prompting him to manually empty an effluent bag (400) which is in fluid communication with the blood treatment apparatus (100) during the blood treatment session in order to receive effluent generated during the treatment session.

4. The control or regulating device (150) according to any one of the preceding claims, being in signal communication with a signal emitter (170) or with a clock of the control or regulating device (150) or an external clock (5000), in particular a clock (160) of the blood treatment apparatus (100), a network clock, a ward clock or an atomic clock, and wherein the control or regulating device (150) is configured, to define as the interruption time point (t1, t2) the time of receiving the signal or reaching the predetermined time when a predetermined signal is received from the signal emitter (170) or when a predetermined time is reached on the clock (160, 5000).

5. The control or regulating device (150) according to any one of the preceding claims, which is programmed to monitor that the effluent bag (400) is not filled beyond a predetermined level or volume.

6. A blood treatment apparatus (100) comprising a control or regulating device (150) according to any one of claims 1 to 5.

7. The blood treatment apparatus (100) according to claim 6, embodied as a dialysis apparatus, hemodialysis apparatus, hemofiltration apparatus or hemodiafiltration apparatus, in particular as an apparatus for chronic renal replacement therapy or for continuous renal replacement therapy (CRRT).

8. An effluent bag draining apparatus (4000), comprising:
- an effluent outlet line (403) for guiding effluent out of an effluent bag (400);
- a pump section (2300) for conveying effluent out of the effluent bag (400);
**characterized by**
a control device (450), programmed to ensure that the pump section (2300) conveys effluent out of the effluent bag (400) when a predetermined draining time point (t3, t4) is reached.

9. The effluent bag draining apparatus (4000) according to claim 8, wherein the control device (450) is programmed to convey effluent out of the effluent bag (400) via the effluent outlet line (403) using the pump section (2300) during a predetermined draining time period (O3, O4).

10. The effluent bag draining apparatus (4000) according to claim 8 or claim 9, wherein the control device (450) is in signal communication with a signal emitter (170) or a clock, in particular a clock (160) of the blood treatment apparatus (100), a clock (460) of the effluent bag draining apparatus (4000), or an external clock (5000), in particular a network clock, a ward clock or an atomic clock,
and wherein the control device (450) is configured to define or set, in relation to this, as the draining time point (t3, t4), the time of receiving the signal or reaching the predetermined time when a predetermined signal from the signal emitter (170) is received or when a predetermined time on the clock is reached.

11. A system comprising a blood treatment apparatus (100) according to any one of claims 6 to 7 and an effluent bag draining apparatus (4000) according to any one of claims 8 to 10.

12. The system according to claim 11, wherein the blood treatment apparatus (100) and the effluent bag draining apparatus (4000) are separate from each other.

13. The system according to claim 11 or 12, wherein the control or regulating device (150) of the blood treatment apparatus (100) and the control device (450) of the effluent bag draining apparatus (4000) are each programmed such that at least one interruption time point (t1, t2) and at least one draining time point (t3, t4) occur simultaneously or offset from each other by a predetermined period of time.

14. The system according to any one of claims 11 to 13, wherein the control or regulating device (150) of the blood treatment apparatus (100) and the control device (450) of the effluent bag draining apparatus(4000) are each programmed such that at least one interruption period (I1, I2) and one draining time period (O3, O4) overlap each other, in particular by at least 50%, 60%, 70%, 80% or 90% of the duration of either of them.

15. The system according to any one of claims 11 to 14, wherein the signal emitter (170) or the clock, with which the control or regulating device (150) of the blood treatment apparatus (100) is in signal communication, and the signal emitter (170) or the clock, with which the control device (450) of the effluent bag draining apparatus (4000) is in signal communication, are identical or synchronized.

## Revendications

1. Un dispositif de commande ou de régulation (150), programmé pour commander ou réguler un appareil de traitement du sang (100) pendant une séance de traitement du sang d'un patient réalisée à l'aide d'un assortiment de tuyaux sanguins extracorporels (300) et de l'appareil de traitement du sang (100), tout en équilibrant des écoulements de liquides, en acheminant au moyen d'une pompe à filtrat (131), en acheminant au moyen d'une pompe à substitut (111) et/ou en acheminant au moyen d'une pompe à liquide de dialyse (121), **caractérisé en ce que** le dispositif de commande ou de régulation (150) est en outre programmé pour :
- interrompre l'équilibrage, et/ou
- interrompre l'écoulement de la solution de calcium ou l'action d'acheminement de la pompe à calcium, l'écoulement de la solution de citrate ou l'action d'acheminement de la pompe à citrate, l'écoulement de la solution d'héparine ou l'action d'acheminement de la pompe à héparine, l'écoulement du filtrat ou l'action d'acheminement de la pompe à filtrat (131), l'écoulement du substitut ou l'action d'acheminement de la pompe à substitut (111) et/ou l'écoulement du liquide de dialyse ou l'action d'acheminement de la pompe à liquide de dialyse (121),
à un ou plusieurs instants d'interruption prédéterminés (t1, t2) pendant la durée de la séance de traitement.

2. Le dispositif de commande ou de régulation (150) selon la première revendication, où l'interruption se produit respectivement pour une durée d'interruption prédéterminée (I1, I2), commençant par l'instant d'interruption correspondant (t1, t2).

3. Le dispositif de commande ou de régulation (150) selon la revendication 1 ou 2, où, lorsque l'instant d'interruption prédéterminé (t1, t2) intervient, une notification ou une alarme peut être transmise à l'utilisateur, l'invitant à vider manuellement une poche à effluent (400) qui est en communication fluidique avec l'appareil de traitement du sang (100) pendant la séance de traitement du sang afin de recueillir l'effluent produit au cours de la séance de traitement.

4. Le dispositif de commande ou de régulation (150) selon l'une des revendications précédentes, étant en communication de signal avec un émetteur de signal (170) ou une horloge du dispositif de commande ou de régulation (150) ou une horloge externe (5000), notamment une horloge (160) de l'appareil de traitement du sang (100), une horloge réseau, une horloge de station ou une horloge atomique, et où le dispositif de commande ou de régulation (150) est configuré pour définir, en tant qu'instant d'interruption (t1, t2), l'instant de réception du signal ou l'atteinte de l'heure prédéterminée lorsqu'un signal prédéterminé provenant de l'émetteur de signal (170) est reçu ou lorsqu'une heure prédéterminée sur l'horloge (160, 5000) est atteinte.

5. Le dispositif de commande ou de régulation (150) selon l'une des revendications précédentes, programmé pour surveiller que la poche à effluent (400) ne soit pas remplie au-delà d'une mesure ou d'un volume prédéterminé(e).

6. Un appareil de traitement du sang (100), comprenant un dispositif de commande ou de régulation (150) selon l'une des revendications 1 à 5.

7. L'appareil de traitement du sang (100) selon la revendication 6, conçu comme un appareil de dialyse, d'hémodialyse, d'hémofiltration ou d'hémodiafiltration, notamment comme un appareil pour la thérapie de remplacement rénal chronique ou continue (CRRT = continuous renal replacement therapy).

8. Un appareil de vidange de poche à effluent (4000), comprenant :
- une conduite d'évacuation d'effluent (403) destinée à évacuer l'effluent hors d'une poche à effluent (400);
- une section de pompage (2300) destinée à acheminer l'effluent hors d'une poche à effluent (400);
**caractérisé par**
un dispositif de commande (450), programmé pour faire en sorte que la section de pompage (2300) achemine l'effluent hors de la poche à effluent (400) lorsqu'un instant de vidange prédéterminé (t3, t4) est atteint.

9. L'appareil de vidange de poche à effluent (4000) selon la revendication 8, où le dispositif de commande (450) est programmé pour acheminer, au moyen de la section de pompage (2300), l'effluent hors de la poche à effluent (400) via la conduite d'évacuation d'effluent (403) pendant une durée de vidange prédéterminée (O3, O4).

10. L'appareil de vidange de poche à effluent (4000) selon la revendication 8 ou 9, où le dispositif de commande (450) est en communication de signal avec un émetteur de signal (170) ou une horloge, notamment une horloge (160) de l'appareil de traitement du sang (100), une horloge (460) du dispositif de vidange de poche à effluent (4000) ou une horloge externe (5000), notamment une horloge réseau, une horloge de station ou une horloge atomique, et où le dispositif de commande (450) est configuré pour définir ou fixer, en fonction de celui-ci, en tant qu'instant de vidange (t3, t4), l'instant de réception du signal ou l'atteinte de l'heure prédéterminée lorsqu'un signal prédéterminé provenant de l'émetteur de signal (170) est reçu ou lorsqu'une heure prédéterminée sur l'horloge est atteinte.

11. Un système comprenant un appareil de traitement du sang (100) selon l'une quelconque des revendications 6 à 7 ainsi qu'un appareil de vidange de poche à effluent (4000) selon l'une quelconque des revendications 8 à 10.

12. Le système selon la revendication 11, où l'appareil de traitement du sang (100) et l'appareil de vidange de poche à effluent (4000) sont disposés séparément l'un de l'autre.

13. Le système selon la revendication 11 ou 12, où le dispositif de commande ou de régulation (150) de l'appareil de traitement du sang (100) et le dispositif de commande (450) de l'appareil de vidange de la poche à effluent (4000) sont chacun programmés de telle sorte qu'au moins un instant d'interruption (t1, t2) et au moins un instant de vidange (t3, t4) se produisent simultanément ou avec un décalage l'un par rapport à l'autre d'une durée prédéterminée.

14. Le système selon l'une des revendications 11 à 13, où le dispositif de commande ou de régulation (150) de l'appareil de traitement du sang (100) et le dispositif de commande (450) de l'appareil de vidange de la poche à effluent (4000) sont chacun programmés de telle sorte qu'au moins une durée d'interruption (I1, I2) et une durée de vidange (03, 04) se chevauchent, notamment à hauteur d'au moins 50 %, 60 %, 70 %, 80 % ou 90 % de la durée de l'une des deux.

15. Le système selon l'une des revendications 11 à 14, où l'émetteur de signal (170) ou l'horloge avec lequel/laquelle le dispositif de commande ou de régulation (150) de l'appareil de traitement du sang (100) est en communication de signal, et l'émetteur de signal (170) ou l'horloge avec lequel/laquelle le dispositif de commande (450) de l'appareil de vidange de la poche à effluent (4000) est en communication de signal, sont identiques ou synchronisé (e) s.
